# EUROPEAN PATENT APPLICATION

(11) **EP 4 613 692 A1**
(43) Date of publication of application: **10.09.2025**
(21) Application number: 23885102.6
(22) Date of filing: 03.11.2023
(51) Int. Cl.: B66C 13/40, B66C 13/18, B66C 13/46, B66C 15/04, B66C 15/06, A61B 5/024

(54) **TOWER CRANE CONTROL SYSTEM**

(30) Priority: 04.11.2022 CN 202211379614
(71) Applicant: Kyland Technology Co., Ltd., Beijing 100041 (CN)
(72) Inventor: GUO, Liping, Beijing 100041 (CN); LV, Zhiyong, Beijing 100041 (CN)
(74) Representative: Stork Bamberger Patentanwälte PartmbB
(86) International application number: PCT/CN2023/129622
(87) International publication number: WO 2024/094178

(57) **Abstract**

A tower crane control system includes a handheld terminal (11) and a tower crane controller (12); the handheld terminal (11) is configured to send hoisting requirements to the crane controller (12) with which a binding relationship has been established in advance, wherein the hoisting requirements include a hoisting target point; the tower crane controller (12) is configured to control hoisting process of an associated target tower crane in a first area according to the hoisting requirements, wherein the hoisting target point is a boundary point of the first area; and the handheld terminal (11) is further configured to control the hoisting process of the target tower crane in a second area when it is determined that the target tower crane has moved to the second area, wherein the hoisting target point is a boundary point of the second area.

## Description

The present application claims the priority benefit of Chinese Patent Application No. 202211379614.6, filed with the China National Intellectual Property Administration on November 04, 2022, the entire content of which is incorporated herein by reference.

### Technical Field

The present application relates to the field of intelligent control technologies, and for instance, to a tower crane control system.

### Background Art

A tower crane is an important hoisting tool in the construction field. The operator in the tower crane's cab (referred to as the tower operator) controls the tower crane to complete the hoisting work of buildings or bridges. This not only has strict requirements for the tower operator's driving and operating skills but also usually results in low work efficiency. In response to the above-mentioned problems, some manufacturers in the industry have implemented remote operation. They transform the control console in the cab at the top of the tower crane to a remote location via communication means, which can be achieved by using a remote cab or a handheld console.

However, the methods for both the remote cab and the handheld console have strict requirements for the tower operator's experience and skills. In particular, the handheld console requires the tower operator to be familiar with the ground environment in advance, and the remote cab requires the construction site to provide a dedicated operating area for the tower operator. Therefore, the methods for the remote operation cannot meet the actual operation requirements of users.

### Summary

The present application provides a tower crane control system to achieve intelligent control of tower cranes.

An aspect of the present application provides a tower crane control system comprising a handheld terminal and a tower crane controller;
the handheld terminal is configured to send hoisting requirements to the tower crane controller with which a binding relationship has been established in advance, wherein the hoisting requirements include a hoisting target point;
the tower crane controller is configured to control hoisting process of an associated target tower crane in a first area according to the hoisting requirements, wherein the hoisting target point is a boundary point of the first area; and
the handheld terminal is further configured to control the hoisting process of the target tower crane in a second area when it is determined that the target tower crane has moved to the second area, wherein the hoisting target point is a boundary point of the second area.

### Brief Description of the Drawings

FIG.1 is a structural schematic diagram of a tower crane control system provided in Embodiment one of the present application.
FIG.2 is an interaction schematic diagram of channel binding provided in Embodiment one of the present application.
FIG.3 is an operation interface schematic diagram of a handheld terminal provided in Embodiment one of the present application.
FIG.4 is an interaction schematic diagram of a tower crane control process provided in Embodiment one of the present application.
FIG.5 is a schematic diagram of division of a hoisting area provided in Embodiment one of the present application.
FIG.6 is an interaction schematic diagram of communication state detection provided in Embodiment two of the present application.

### Detail Description

The technical solutions in the embodiments of the present application will be described with reference to the accompanying drawings in the embodiments of the present application in a clear and complete manner. The described embodiments are merely a part of the embodiments of the present application.

Terms such as "first" and "second" in the description, the claims, and the above-mentioned drawings of the present application are used to distinguish similar objects and are not intended to describe a specific order or sequence. It could be appreciated that such data can be interchanged under appropriate circumstances so that the embodiments of the present application described herein can be implemented in an order other than those illustrated or described herein. In addition, terms such as "include" and "have", and any variations thereof, are intended to cover non-exclusive inclusion. For example, a process, a method, a system, product, or a device that includes a series of steps or units is not necessarily limited to those steps or units listed but may include other steps or units not listed or inherent to such process, method, product, or device.

### Embodiment One

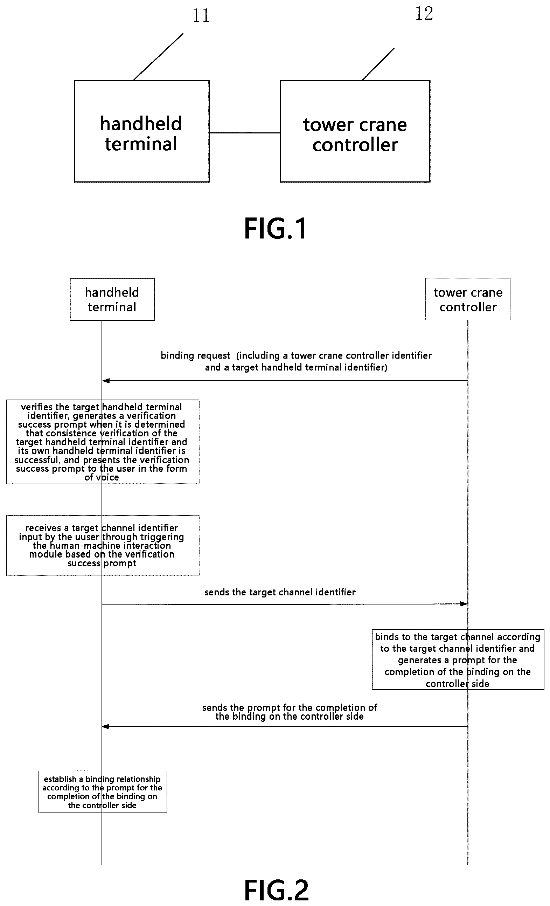

FIG.1 is a structural schematic diagram of a tower crane control system provided in Embodiment one of the present application. This embodiment is applicable to a situation in which a tower crane is controlled. As shown in FIG. 1, the system includes a handheld terminal 11 and a tower crane controller 12.

The handheld terminal 11 is configured to send hoisting requirements to the tower crane controller 12 with which a binding relationship has been established in advance, wherein the hoisting requirements include a hoisting target point. The tower crane controller 12 is configured to control hoisting process of an associated target tower crane in a first area according to the hoisting requirements, wherein the hoisting target point is a boundary point of the first area. The handheld terminal 11 is further configured to control hoisting process of the target tower crane in a second area when it is determined that the target tower crane has moved to the second area, wherein the hoisting target point is a boundary point of the second area. In this embodiment, the hoisting target point may be a lower boundary point of the first area and also an upper boundary point of the second area. The hoisting target point may be a position or area at a certain height from a position at which the hoisted item is located or a position at which a user who raises the hoisted item via the handheld terminal is located, or a position or area at a certain height from a position at which the hoisted item is lowered or a position at which a user who lowers the hoisted item via the handheld terminal is located.

In this embodiment, the hoisting target point may be a demarcation point having control right of the tower crane and corresponding to the handheld terminal. When a hook of the tower crane moves downward, before the hook reaches the hoisting target point, the tower crane controller plans a path and automatically controls operation of the hook of the target tower crane according to the planned path. After the hook passes through the hoisting target point, the operation of the hook of the target tower crane may be directly controlled by the handheld terminal at a slow speed.

The handheld terminal includes an initiation authentication module. The handheld terminal is configured to receive an access code through the initiation authentication module and start according to the access code.

In order to prevent an illegal user from using the handheld terminal without authorization, the access code of the user may be received by the initiation authentication module, and a corresponding standard access code may be preset for each handheld terminal. Only when the access code entered by the user is consistent with the standard access code of the accessed handheld terminal, the handheld terminal will be started. If the access code entered by the user is inconsistent with the standard access code of the accessed handheld terminal, the handheld terminal will be in the off state. The initiation authentication module in this embodiment may also be a key-operated switch. Only when the user holds a key corresponding to the accessed handheld terminal and triggers the key-operated switch with the corresponding key, the handheld terminal can be started. From the above, it can be seen that the initiation authentication module essentially authenticates the identity of the accessing user, thus avoiding hoisting work risks due to random operation of the tower crane controller by illegal users.

The handheld terminal is further configured to send control instructions to the bound tower crane controller through a target channel in an activated state, wherein different channels correspond to different tower crane controllers respectively.

The handheld terminal is further configured to establish a binding relationship with the tower crane controller and activate the target channel through a trigger operation of target channel human-machine interaction module on an operation interface of the handheld terminal by a user, wherein the operation interface of the handheld terminal includes at least one channel human-machine interaction module, and different channel human-machine interaction modules correspond to different tower crane controllers respectively.

The operation interface of the handheld terminal further includes indication information associated with the at least one channel human-machine interaction module. The handheld terminal is further configured to update the indication information associated with the target channel human-machine interaction module to a first state when it is determined that the target channel associated with the target channel human-machine interaction module is in the activated state, wherein the first state indicates that the target channel is in the activated state.

In this embodiment, before the handheld terminal and the tower crane controller control the hoisting of the target tower crane in different areas respectively, a binding relationship is required to be established with the tower crane controller in advance, and the binding relationship between them is established through channel binding. FIG.2 is an interaction schematic diagram of channel binding in this embodiment. As shown in FIG.2, when the tower crane controller receives a binding instruction from the background maintenance personnel, it generates a binding request. The handheld terminal thus receives the binding request sent by the tower crane controller. The binding request includes a tower crane controller identifier and a target handheld terminal identifier, wherein the target handheld terminal identifier is the identifier of the handheld terminal with which the binding relationship is to be established, which has been entered by the background maintenance personnel in the tower crane controller in advance. The handheld terminal obtains a target channel identifier according to the binding request and performs channel binding with the tower crane controller based on the target channel identifier to establish the binding relationship. Therefore, in this embodiment, the handheld terminal and the tower crane controllers establish binding relationships through channels, wherein different channels correspond to different tower crane controllers.

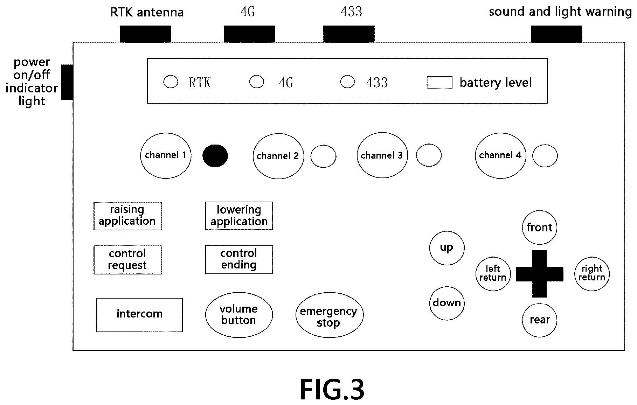

That the handheld terminal obtains the target channel identifier according to the binding request is achieved through operation of the operation interface of the handheld terminal by the user. FIG.3 is an operation interface schematic diagram of the handheld terminal in this embodiment. As shown in FIG.3, the handheld terminal verifies the target handheld terminal identifier in the binding request. When it is determined that consistence verification of the target handheld terminal identifier and its own handheld terminal identifier is successful,the handheld terminal generates a verification success prompt and presents it to the user in the form of voice. There are multiple channel keys on the handheld terminal. The user may select a channel by triggering a combination key composed of the target channel key and the control request key according to the prompt. After the handheld terminal obtains the target channel identifier according to the operation of the user, it sends the target channel identifier determined on the user side to the tower crane controller that matches the tower crane controller identifier.

In this embodiment, one handheld terminal may be bound to multiple tower crane controllers at the same time. In order to ensure communication with the specified tower crane controller and control the hoisting work of the target tower crane through the specified tower crane controller, the user may trigger the target channel human-machine interaction module on the operation interface of the handheld terminal. The interaction module in this embodiment may be in the form of a key. However, the form of the interaction module is not limited in this embodiment. For example, the target channel human-machine interaction module may be a target channel key. Since different channel keys correspond to different tower crane controllers, the handheld terminal generates a channel activation request according to the trigger operation of the user and performs channel activation interaction with the tower crane controller matched with the target channel key based on the channel activation request, thereby activating the target channel. Only when the target channel is activated, the instructions generated through triggering of the operation keys of the handheld terminal by the user may be transmitted to the target tower crane controller through the activated target channel. For example, if the user wants to control a tower crane associated with a tower crane controller 1, since a key for channel 1 corresponds to the the tower crane controller 1, which correspondence has been determined during the previous binding, and since the channel is selected on the user side, which means that the user knows the correspondence between the human-machine interaction module and the tower crane controller, the user presses the key for channel 1, and the terminal generates an activation request for the channel 1 according to triggering of the channel key for the channel 1 by the user. The activation request for the channel includes the handheld terminal identifier and the target tower crane controller identifier.

The handheld terminal in this embodiment further includes indication information associated with the channel keys, such as indicator lights, and states of the channels are indicated by opening of the indicator lights. When it is determined that the target channel associated with the target channel key is in the activated state, by switching the indicator light to a constantly lit state, it is convenient for the user to timely and intuitively know which tower crane controller is currently controlled by the handheld terminal. **In** addition, when the channel activation request generated through triggering of the target channel key by the user is received, the indicator light associated with the target channel key may further be switched to a flashing state. It could be appreciated that the indication information in this embodiment may further be sound, and different states of the target channel may be indicated by sounds with different contents. Therefore, the form of the indication information is not limited in this embodiment. In this embodiment, the activation states of multiple channels are indicated by using indication information, thereby presenting it more intuitive to the user to notify the user which channel is currently in an activated state and the working state of the tower crane controller bound to the activated channel.

The handheld terminal further includes request interaction modules, wherein the request interaction modules include a raising application interaction module and a lowering application interaction module. The handheld terminal is further configured to: start a positioning module for automatic positioning to obtain the hoisting target point according to trigger operation of the raising application interaction module or the lowering application interaction module by a user; obtain an identifier of the tower crane controller with which the binding relationship has been established in advance and its own handheld terminal identifier; and generate the hoisting requirements based on the hoisting target point, the identifier of the tower crane controller, and the handheld terminal identifier.

The tower crane controller is configured to control the hoisting process of the associated target tower crane in the first area according to the hoisting requirements in such a way of determining a planned path according to the hoisting requirements and controlling the target tower crane to reach the hoisting target point in the first area along the planned path.

The handheld terminal is further configured to send a permission acquisition request to the tower crane controller, the tower crane controller is further configured to generate a permission transfer instruction according to the permission acquisition request and send the permission transfer instruction to the handheld terminal, and the handheld terminal is configured to control the hoisting process of the target tower crane in the second area in such a way of obtaining a control right for the target tower crane according to the permission transfer instruction and sending operation instructions to the target tower crane based on the control right after the target tower crane reaches the second area, so that the target tower crane performs the hoisting workaccording to the operation instructions by taking the hoisting target point as a starting point.

The operation interface of the handheld terminal further includes a control request interaction module. The handheld terminal is further configured to generate a permission acquisition request according to a trigger operation of the control request interaction module by the user and send the permission acquisition request to the tower crane controller.

The operation interface of the handheld terminal further includes a control operation interaction module. The handheld terminal is further configured to generate a key code according to an trigger operation of the control operation interaction module by the user and send the key code as an operation instruction to the target tower crane controller via wireless communication. The target tower crane controller is further configured to control the hoisting work of the target tower crane according to the operation instruction by taking the hoisting target point as a starting point.

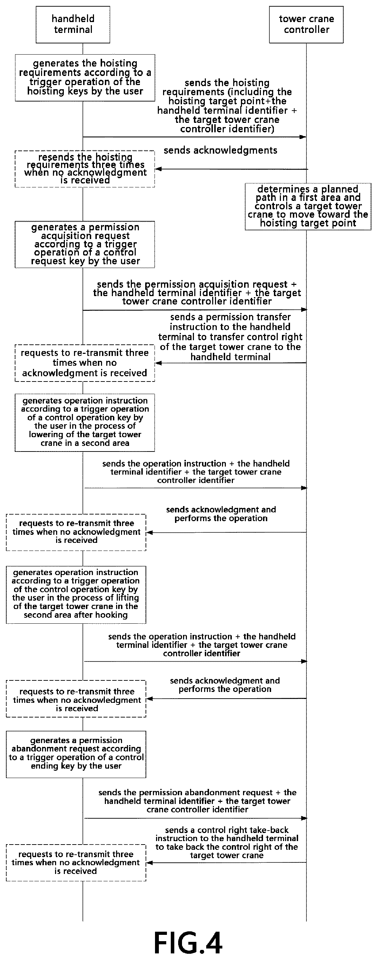

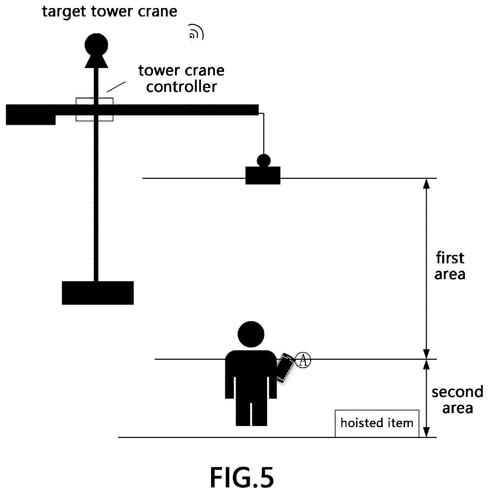

FIG.4 is an interaction schematic diagram of the tower crane control process in this embodiment. After the handheld terminal completes channel binding and establishes a connection with the tower crane controller through channel selection, control process of the tower crane may be implemented with reference to FIG.4. The interaction module in this embodiment may be in the form of a key. However, the form of the interaction module is not limited in this embodiment. As shown in FIG.3, the operation interface of the handheld terminal includes hoisting keys such as a raising application key, a lowering application key and the like. When the user presses the hoisting keys, since the handheld terminal has an automatic positioning function, the positioning module will be triggered for automatic positioning to obtain the hoisting target point. For example, when the user presses the raising application key and needs to hoist a nearby hoisted item, the handheld terminal will obtain the tower crane controller identifier of the target tower crane controller bound to the target channel and its own handheld terminal identifier according to the binding relationship, generate the hoisting requirements based on the hoisting target point, the tower crane controller identifier and the handheld terminal identifier, and send the hoisting requirements to the tower crane controller with which the communication connection has been established. When it is determined that no response information fed back by the tower crane controller is received, the handheld terminal will re-transmit multiple times to ensure that the tower crane controller can accurately receive the message sent by the handheld terminal. The operation area of the hook before the handheld terminal obtains the control right is referred to the first area. The area from the operation position point A of the hook when the control right is obtained to the position at which the hoisted item is located, that is, the hoisting target point, is referred to the second area. The point A is taken as the lower boundary point of the first area, and at the same time, the operation position point A of the hook when the control right is obtained is taken as the upper boundary point of the second area. FIG.5 is a schematic diagram of division of the hoisting area in this embodiment. As shown in FIG.5, the tower crane controller determines a planned path according to the hoisting target point in the hoisting requirements and controls the target tower crane to move towards the hoisting target point along the planned path in the first area.

In the process that the tower crane controller controls movement of the target tower crane in the first area, when the hook is still at a certain distance from the hoisting target point, in order to accurately hang the hoisted item on the target tower crane, the user may press the control request key on the handheld terminal to send the permission acquisition request to the tower crane controller, so as to achieve fine tuning of the hoisting work of the target tower crane in the second area through triggering of the operation key on the handheld terminal by the user. For example, a down key code is generated according to an trigger operation of the down key by the user, and the down key code is used as a down operation instruction. The down operation instruction is sent to the tower crane controller via wireless communication, and the tower crane controller controls the target tower crane to move downward according to the obtained down operation instruction. When the target tower crane moves to the position at which the hoisted item is located, the user may manually operate to hook the hoisted item onto the target tower crane to complete the raising operation of the hoisted item. After hooking the hoisted item onto the target tower crane, the user may continue to control the target tower crane to lift in the second area by operating the handheld terminal, and finally guide the target tower crane out of the second area through fine tuning.

When the handheld terminal sends the operation instruction to the tower crane controller, it also sends the handheld terminal identifier and the target tower crane controller identifier bound to the target channel to the tower crane controller. The function of sending the handheld terminal identifier is to inform the tower crane controller which handheld terminal sent the current permission acquisition request, and the function of sending the target tower crane controller identifier is to facilitate the verification of the tower crane controller to determine whether the permission acquisition request is sent to itself. It can be seen that every time the handheld terminal sends a message to the tower crane controller, it sends its own identifier and the target tower crane controller identifier together for the tower crane controller to verify, ensuring that the message is accurately sent to the target party. In addition, when it is determined thatno response for the massage sent by the handheld terminal is received from the tower crane controller, it re-sends the massage a specified number of times to ensure that the tower crane controller can accurately receive the message sent by the handheld terminal.

The operation interface of the handheld terminal further includes a control ending interaction module. The handheld terminal is further configured to generate a permission abandonment request according to a trigger operation of the control ending interaction module by the user and send the permission abandonment request to the tower crane controller. The tower crane controller is further configured to take back control right of the target tower crane according to the permission abandonment request.

The operation interface of the handheld terminal further includes an emergency stop operation interaction module. The handheld terminal is further configured to generate an emergency stop instruction according to a trigger operation of a key for the emergency stop interaction module by a user and send the emergency stop instruction to the tower crane controller. The tower crane controller is further configured to terminate the hoisting work of the target tower crane according to the emergency stop instruction.

When the target tower crane moves out of the second area or the user needs to abandonan the control of the target tower crane due to temporary situations during the operation in the second area, the user may trigger a control ending key on the operation interface to take back the control right of the target tower crane. In addition, when the handheld terminal controls the target tower crane to perform the hoisting in the second area, if an obstacle is found in the front of the traveling path and there is a collision risk, the user may press an emergency stop operation key. The handheld terminal generates an emergency stop instruction according to a trigger operation of the emergency stop operation key by the user and sends the emergency stop instruction to the tower crane controller. The tower crane controller terminates the hoisting work of the target tower crane according to the emergency stop instruction, thus ensuring the safety of the hoisting work.

The tower crane controller is further configured to generate a warning information when there is a risk of obstacle collision in the process of controlling the target tower crane to move towards the hoisting target point along the planned path, and send the warning information to a warning module of the handheld terminal by using a long-range radio (LoRa) communication mode. The handheld terminal is further configured to alarm in a specified manner according to the warning information through the warning module, wherein the specified manner includes voice or light.

In the process of controlling the tower crane to move along the planned path, if an emergency situation is detected in the front of the planned path, such as an unknown obstacle ahead and the tower crane controller is unable to effectively control the tower crane to avoid the obstacle, the tower crane controller will generate a warning information and send the generated warning information to the handheld terminal by using the LoRa communication mode. The handheld terminal then makes sound and light warning according to the warning information. As shown in FIG.3, the operation interface of the handheld terminal includes an indicator light and a microphone for acoustic and optical warning. The handheld terminal may analyze the danger level of the warning information and adopt different alarm methods according to different danger levels. For example, when it is determined that the danger level is low, the warning module controls the indicator light to flash and the microphone to make a low-frequency voice alarm; when it is determined that the danger level is high, the warning module controls the indicator light to be in a constantly lit state and the microphone to make a high-frequency voice alarm. This is merely an example in this embodiment, and the alarm methods used are not limited. When the user receives the alarm signals, corresponding measures may be taken timely to avoid hoisting risks as far as possible.

The handheld terminal further includes an intercom interaction module. The handheld terminal is further configured to start the intercom interaction module according to a trigger operation of the intercom interaction module by a user, receive a first voice information input by the user to whom the terminal belongs through the intercom interaction module, and send the first voice information to specified terminal(s) by using a 433MHz digital communication mode. The handheld terminal is further configured to receive a second voice information sent by the specified terminal(s) by using the 433 MHz digital communication mode and play the second voice information.

The communication among the tower crane command members at the construction site, including communication among signal workers, between the signal workers and the tower operator, and between the signal workers and other command members, may be achieved through the intercom function for direct communication and collaborative work. For example, when a user presses an intercom key on the human-machine interaction interface shown in FIG.3, the intercom interaction module is triggered and activated. The intercom interaction module in the activated state may receive the first voice information from the user and send the first voice information to the specified terminal(s) by using the 433 MHz digital communication mode. At the same time, the handheld terminal may also receive, through the intercom interaction module, the second voice information sent by the specified terminal(s) by using the 433 MHz digital communication mode and play the second voice information. Here, the specified terminal(s) refers to terminal(s) in the same working frequency band as the handheld terminal of the user. The number of specified terminals is not limited in this embodiment. That is, the may communicate with multiple tower crane command members simultaneously through the intercom interaction module to improve the efficiency of the hoisting work.

In this embodiment of the present application, the hoisting requirements are sent to the tower crane controller by the handheld terminal without the need for special scenarios and strict requirements for user skills, enabling the tower crane controller and the handheld terminal to control the target tower crane in different areas respectively to complete the hoisting work, which improves the operating efficiency of the tower crane and meets the actual operating requirements of users.

### Embodiment Two

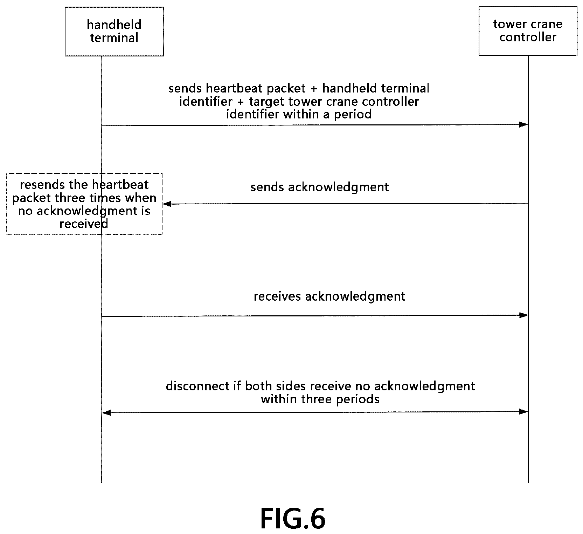

FIG.6 is an interaction schematic diagram of communication state detection provided in Embodiment two of the present application. Based on the above-mentioned embodiment, this embodiment detects the communication state between the handheld terminal and the tower crane controller through the heartbeat mechanism.

The handheld terminal is further configured to regularly send a heartbeat packet to the tower crane controller through the target channel, wherein the heartbeat packet includes the handheld terminal identifier. The tower crane controller is further configured to generate a response packet according to the heartbeat packet and feedback the response packet to the handheld terminal when it receives the heartbeat packet within a specified period, and determine that the handheld terminal fails and take back the control right of the target tower crane when it does not receive the heartbeat packet within the specified period.

In one implementation, when the handheld terminal is working normally, it sends the heartbeat packet to the tower crane controller with the specified period. At the same time of sending the heartbeat packet, the handheld terminal further sends the handheld terminal identifier and the target tower crane controller identifier to the tower crane controller. When the tower crane controller determines that it has received the heartbeat packet, it may determine that the communication state of the channel bound with the handheld terminal is good. The tower crane controller further generates the response packet according to the heartbeat packet and feedback the response packet to the handheld terminal. Since the handheld terminal may be bound to multiple tower crane controllers, the response packet includes the target tower crane controller identifier to inform which tower crane controller the current response packet is fed back by.

In another implementation, when the handheld terminal fails, it stops sending the heartbeat packet to the tower crane controller. When the tower crane controller determines that it has not received the heartbeat packet within the specified time range, it can determine that the communication with the handheld terminal is abnormal. To ensure the normal operation of the target tower crane, the tower crane controller takes back the control right of the target tower crane, and may switch the control method of the control right to the control by the tower operator. In this embodiment, the transfer method of the control right of the target tower crane when the heartbeat packet is abnormal is not limited. As long as the normal operation of the target tower crane can be ensured, it is within the protection scope of the present application and is not limited in this embodiment.

In this embodiment of the present application, the hoisting requirements are sent to the tower crane controller by the handheld terminal without the need for special scenarios and strict requirements for user skills, enabling the tower crane controller and the handheld terminal to control the target tower crane in different areas respectively to complete the hoisting task, which improves the operating efficiency of the tower crane and meets the actual operating requirements of users. Through the heartbeat mechanism of the communication state detection module, the communication state between the handheld terminal and the tower crane controller is detected in real time. When the terminal is connected, the transfer of the control right of the tower crane is carried out immediately, ensuring the normal execution of the hoisting work.

The steps may be reordered, added or deleted by using the processes in various forms shown above. For example, the multiple steps described in the present application may be executed in parallel, sequentially, or in a different order. As long as the results expected by the technical solutions of the present application can be achieved, no limitation is made herein.

## Claims

1. A tower crane control system, **characterized by** comprising a handheld terminal (11) and a tower crane controller (12);
the handheld terminal (11) is configured to send hoisting requirements to the tower crane controller (12) with which a binding relationship has been established in advance, wherein the hoisting requirements include a hoisting target point;
the tower crane controller (12) is configured to control hoisting process of an associated target tower crane in a first area according to the hoisting requirements, wherein the hoisting target point is a boundary point of the first area; and
the handheld terminal (11) is further configured to control the hoisting process of the target tower crane in a second area when it is determined that the target tower crane has moved to the second area, wherein the hoisting target point is a boundary point of the second area.

2. The system according to claim 1, **characterized in that** the tower crane controller (12) is configured to control the hoisting process of the associated target tower crane in the first area according to the hoisting requirements in such a way of determining a planned path according to the hoisting requirements and controlling the target tower crane to reach the hoisting target point in the first area along the planned path.

3. The system according to claim 1, **characterized in that** the handheld terminal (11) is further configured to send a permission acquisition request to the tower crane controller (12);
the tower crane controller (12) is further configured to generate a permission transfer instruction according to the permission acquisition request and send the permission transfer instruction to the handheld terminal (11); and
the handheld terminal (11) is configured to control the hoisting process of the target tower crane in the second area in such a way of obtaining a control right for the target tower crane according to the permission transfer instruction and sending operation instructions to the target tower crane based on the control right after the target tower crane reaches the second area, so that the target tower crane performs the hoisting work according to the operation instructions by taking the hoisting target point as a starting point.

4. The system according to claim 1, **characterized in that** the handheld terminal (11) is further configured to send control instructions to the bound tower crane controller (12) through a target channel in an activated state, wherein different channels correspond to different tower crane controllers (12) respectively.

5. The system according to claim 4, **characterized in that** the handheld terminal (11) is further configured to establish the binding relationship with the tower crane controller (12) and activate the target channel through a trigger operation of a target channel human-machine interaction module on an operation interface of the handheld terminal (11) by a user, wherein the operation interface of the handheld terminal (11) includes at least one channel human-machine interaction module, and different channel human-machine interaction modules correspond to different tower crane controllers (12) respectively.

6. The system according to claim 5, **characterized in that** the operation interface of the handheld terminal (11) further includes indication information associated with the at least one channel human-machine interaction module; and
the handheld terminal (11) is further configured to update the indication information associated with the target channel human-machine interaction module to a first state when it is determined that the target channel associated with the target channel human-machine interaction module is in the activated state, wherein the first state indicates that the target channel is in the activated state.

7. The system according to claim 4, **characterized in that** the handheld terminal (11) includes hoisting request interaction modules, wherein the hoisting request interaction modules include a raising application interaction module and a lowering application interaction module; and
the handheld terminal (11) is further configured to: start a positioning module for automatic positioning to obtain the hoisting target point according to a trigger operation of the raising application interaction module or the lowering application interaction module by a user; obtain an identifier of the tower crane controller (12) with which the binding relationship has been established in advance and its own handheld terminal identifier; and generate the hoisting requirements based on the hoisting target point, the identifier of the tower crane controller (12), and the handheld terminal identifier.

8. The system according to claim 4, **characterized in that** the operation interface of the handheld terminal (11) includes a control request interaction module; and
the handheld terminal (11) is further configured to generate a permission acquisition request according to a trigger operation of the control request interaction module by a user and send the permission acquisition request to the tower crane controller (12).

9. The system according to claim 8, characterized in thatthe operation interface of the handheld terminal (11) further includes a control ending interaction module;
the handheld terminal (11) is further configured to generate a permission abandonment request according to a trigger operation of the control ending interaction module by the user and send the permission abandonment request to the tower crane controller (12); and
the tower crane controller (12) is further configured to take back control right of the target tower crane according to the permission abandonment request.

10. The system according to claim 4, **characterized in that** the operation interface of the handheld terminal (11) includes a control operation interaction module;
the handheld terminal (11) is further configured to generate a key code according to a trigger operation of the control operation interaction module by a user and send the key code as an operation instruction to the tower crane controller (12) via wireless communication; and
the tower crane controller (12) is further configured to control the hoisting work of the target tower crane according to the operation instruction by taking the hoisting target point as a starting point.

11. The system according to claim 2, **characterized in that** the handheld terminal (11) includes a warning module;
the tower crane controller (12) is further configured to generate a warning information when there is a risk of obstacle collision in the process of controlling the target tower crane to move towards the hoisting target point along the planned path, and send the warning information to the warning module of the handheld terminal (11) by using a long-range radio (LoRa) communication mode; and
the handheld terminal (11) is further configured to alarm in a specified manner according to the warning information through the warning module, wherein the specified manner includes voice or light.

12. The system according to claim 11, **characterized in that** the operation interface of the handheld terminal (11) further includes an emergency stop operation interaction module;
the handheld terminal (11) is further configured to generate an emergency stop instruction according to a trigger operation of the emergency stop operation interaction module and send the emergency stop instruction to the tower crane controller (12); and
the tower crane controller (12) is further configured to terminate the hoisting work of the target tower crane according to the emergency stop instruction.

13. The system according to claim 1, **characterized in that** the operation interface of the handheld terminal (11) includes an intercom interaction module;
the handheld terminal (11) is further configured to start the intercom interaction module according to a trigger operation of the intercom interaction module by a user, receive a first voice information input by the user to whom the terminal belongs through the intercom interaction module, and send the first voice information to specified terminal(s) by using a 433MHz digital communication mode; and
the handheld terminal (11) is further configured to receive a second voice information sent by the specified terminal(s) by using the 433MHz digital communication mode and play the second voice information.

14. The system according to claim 4, **characterized in that** the handheld terminal (11) is further configured to regularly send a heartbeat packet to the tower crane controller (12) through the target channel, wherein the heartbeat packet includes the handheld terminal identifier; and
the tower crane controller (12) is further configured to generate a response packet according to the heartbeat packet and feedback the response packet to the handheld terminal (11) when it receives the heartbeat packet within a specified period, and determine that the handheld terminal (11) fails and take back the control right of the target tower crane when it does not receive the heartbeat packet within the specified period.

15. The system according to claim 1, **characterized in that** the handheld terminal (11) includes an initiation authentication module; and
the handheld terminal (11) is further configured to receive an access code through the initiation authentication module and start according to the access code.
